Europäisches Patentamt

European Patent Office

Office européen des brevets

(19)

(11) Publication number: **0 308 962 B1**

(12) **EUROPEAN PATENT SPECIFICATION**

(45) Date of publication of patent specification: **23.12.92**   (51) Int. Cl.⁵: **C10G  45/68**, C10G 67/06

(21) Application number: **88115698.8**

(22) Date of filing: **23.09.88**

(54) Process for production of dimethylnaphthalenes.

(30) Priority: **24.09.87 JP 237428/87**

(43) Date of publication of application:
**29.03.89 Bulletin  89/13**

(45) Publication of the grant of the patent:
**23.12.92 Bulletin  92/52**

(84) Designated Contracting States:
**DE ES IT**

(56) References cited:
**EP-A- 0 124 328          EP-A- 0 164 905
GB-A- 960 164             US-A- 3 055 956
US-A- 3 153 675           US-A- 3 249 644**

**HYDROCARBON PROCESSING, vol. 59, no. 5,
May 1980, pages 110-114, Gulf Publishing
Co., Houston, Texas, US; M.F. SYMONIAK:
"Upgrade naphtha to fuels and feedstocks"**

(73) Proprietor: **NIPPON MINING COMPANY LIMIT-
ED
12-32, Akasaka 1-chome Minato-ku
Tokyo(JP)**

(72) Inventor: **Yano, Kyoji, c/o Nippon Mining Co.
Ltd.
Mizushima Oil Refinary, 1, Ushiodori
2-chome
Kurashiki-shi, Okayama(JP)**
Inventor: **Aizawa, Shirou, c/o Nippon Mining
Co. Ltd.
Mizushima Oil Refinary, 1, Ushiodori
2-chome
Kurashiki-shi, Okayama(JP)**

(74) Representative: **Hansen, Bernd, Dr.rer.nat. et
al
Hoffmann, Eitle & Partner Patentanwälte Ar-
abellastrasse 4 Postfach 81 04 20
W-8000 München 81(DE)**

## Description

### FIELD OF THE INVENTION

The present invention relates to a process for production of dimethylnaphthalenes from a raffinate resulting from recovery of normal paraffins from a hydrodesulfurized kerosene fraction (i.e. the normal paraffin depleted fraction).

### BACKGROUND OF THE INVENTION

2,6- or 2,7-naphthalenedicarboxylic acid obtained by oxidation of dimethylnaphthalenes, particularly 2,6- or 2,7-dimethylnaphthalene, is used as a starting material for production of polyesters such as polyethylene naphthalates. These polyesters provide synthetic fibers and films having excellent characteristics. It has therefore been desired to develop a process for producing 2,6- or 2,7-dimethylnaphthalene at low costs and with high quality.

Dimethylnaphthalenes are contained in coal tar or a cycle oil in the fluid catalytic cracking process and, thus, a method of recovering dimethylnaphthalenes by distillation of coal tar or the cycle oil has been proposed (see, for example, JP-A-60-69042 (the term "JP-A" as used herein means an "unexamined published Japanese patent application")).

In the method of recovering dimethylnaphthalenes from coal tar or the cycle oil in the fluid catalytic cracking process since the cycle oil contains a high concentration of nitrogen and sulfur compounds, the dimethylnaphthalenes obtained are contaminated with these nitrogen and sulfur compounds. These nitrogen and sulfur compounds accelerate catalyst poisoning in isomerization of substituted dimethylnaphthalenes other than 2,6- or 2,7-dimethylnaphtha-lene, and further in adsorption separation of 2,6- or 2,7-dimethylnaphthalene by the use of a zeolite and so on. It is therefore necessary to decrease the amounts of nitrogen and sulfur compounds in dimethylnaphthalenes to about 10 ppm or less. Concerning hydrotreating to decrease the amounts of the nitrogen and sulfur compounds to about 10 ppm or less, it should be carried out under severe conditions. Hydrotreating under such severe conditions inevitably causes hydrogenation and cracking of dimethylnaphthalenes, resulting in a great reduction in yield of dimethylnaphthalenes. Thus, additional dehydrogenation is needed, and a problem arises in that the production cost is markedly increased.

Normal paraffins are recovered from a kerosene fraction as a starting material for production of linear alkylbenzene sulfonates (LAS) as synthetic detergents.

As a result of investigations, it has been found that a product oil obtained by reforming reaction of the above-described raffinate contains a large amount of dimethylnaphthalenes and that the product is almost free from nitrogen and sulfur compounds.

It is reported that catalytic reforming of a kerosene fraction provides heavy aromatic compounds and that the heavy aromatic compounds contain dimethylnaphthalenes (Sekiyu Gakkaishi, Vol. 13, No. 6 (1970), pp. 468-474). But, astonishingly, by the reforming reaction of the raffinate, dimethylnaphthalenes can be formed inan amount of about 1.5 times that in the reforming reaction of the kerosene fraction.

### SUMMARY OF THE INVENTION

An object of the present invention provides a process in which dimethylnaphthalenes with a low content of sulfur and nitrogen compounds can be produced in high yield.

That is, the present invention relates to a process for producing dimethylnaphthalenes which comprises subjecting a raffinate resulting from recovery of normal paraffins from a hydrodesulfurized kerosene fraction (i.e. the normal paraffin depleted fraction) to reforming reaction and then recovering dimethylnaphthalenes from the product oil.

### DETAILED DESCRIPTION OF THE INVENTION

The hydrodesulfurized kerosene fraction as referred to herein is a kerosene fraction obtained by atmospheric distillation of a crude oil or cracking oil, etc., generally a distillate within the boiling range of from 150 to 300°C, which has been subjected to desulfurization under the commonly used hydrodesulfurization conditions, for example, with catalysts prepared by supporting at least one of cobalt, nickel, molybdenum, and tungsten on a carrier such as alumina or silica-alumina and under conditions of temperature range of from 280 to 430°C, pressure range of from 10 to 200 $\overline{kg}/cm^2$ (981 to 19612 kPa)

2

liquid hourly space velocity (LHSV) range of from 0.5 to 15 $h^{-1}$, and hydrogen recycle amount range of from 70 to 2,400 $Nm^3/kl$. A kerosene fraction with reduced sulfur and nitrogen contents to about 50 ppm or less is preferably used.

In the present invention, a raffinate resulting from the recovery of normal paraffins from the above-described hydrodesulfurized kerosene fraction is used. This recovery of normal paraffins is preferably carried out by adsorption separation using a molecular sieve, such as by the Iso-Siv method (cf. Hydrocarbon Processing, 59, No. 5, May, 1980, pp. 110-114), the Molex method (cf. D.B.Broughton et al., Petrol. Refiner., 40(5), 173 (1961), and the BP method (cf. A.A. Yeo et al., Six World Petroleum congress, Sect. IV-Paper 15 (1963)). As the raffinates, those in which at least 50% by weight, particularly from 70 to 95% by weight, of normal paraffins in the kerosene fraction are recovered are preferred from the viewpoint of yield of dimethylnaphthalenes. In this case, the order of the hydrodesulfurization and the recovery of normal paraffins is not critical. It is, however, preferred from the viewpoint of catalyst poison of zeolite that the hydrodesulfurization is first carried out.

For the reforming reaction, a catalytic reforming process which is widely used for production of high-octane value gasoline from a naphtha fraction and so forth can be employed. This can be carried out by the use of, e.g., a catalyst prepared by supporting platinum alone or in combination with rhenium, germanium, tin, iridium, or ruthenium on a carrier of alumina and under conditions of temperature range of from 400 to 550°C, pressure range of from 1 to 100 $kg/cm^2$ (98 to 9807 kPa), liquid hourly space velocity (LHSV) range of from 0.1 to 3 $h^{-1}$, and hydrogen/oil molar ratio range of from 0.5 to 20.

In another embodiment, the reforming reaction can be carried out by the use of a zeolite, or crystalline aluminosilicate, silica, alumina, zirconia, titania, chromia, solid phosphoric acid, or oxides of indium, lanthanum, manganese, cerium or tin, or acidic refractories containing a mixture of two or more thereof, or catalysts prepared by containing therein or supporting thereon metals such as platinum, palladium, and rhenium and under conditions of temperature range of from 250 to 700°C, pressure range of from 1 to 100 $kg/cm^2$ (98 to 9807 kPa), LHSV range of from 0.1 to 20 $h^{-1}$, and hydrogen/oil molar ratio range of from 0.5 to 20.

The product oil after the reforming reaction contains a relatively high concentration of dimethylnaphthalenes, and the dimethylnaphthalenes are recovered by techniques such as distillation, solvent extraction, conventional crystallization, high-pressure crystallization (cf. Kagaku Kogaku, 51, No. 6, 428-433 (1987)), and combinations thereof. The recovery by distillation is preferred from the economic standpoint, and by collecting a 255-270°C fraction, a high concentration of dimethylnaphthalenes can be obtained.

2,6- and 2,7-dimethylnaphthalnes are separated and recovered from the dimethylnaphthalenes by known techniques such as the adsorption separation method using a zeolite, the crystallization method, and the separation method through the formation of a complex compound. The residue after the recovery is isomerized by the use of an isomerization catalyst and recycled for the above-described separation and recovery.

In accordance with the present invention, dimethylnaphthalenes are recovered from a product oil resulting from the reforming reaction of a hydrodesulfurized kerosene fraction. Thus, dimethylnaphthalenes with a low content of sulfur and nitrogen compounds can be produced in quite high yield.

The present invention is described in greater detail with reference to the following examples.

## EXAMPLES 1 AND 2, AND COMPARATIVE EXAMPLE 1

A desulfurized kerosene fraction having properties shown in Table 1, as obtained by hydrodesulfurization of a kerosene fraction, and a raffinate having properties as shown in Table 1, as obtained by recovering 90% by weight of normal paraffins from the above-described kerosene fraction by the use of a molecular sieve, were used as starting materials and subjected to reforming reaction by the use of a catalytic reforming catalyst comprising an alumina carrier having supported thereon 0.2% by weight of platinum and under the conditions as shown in Table 2. Properties of the product oil and the dimethylnaphthalene content are shown in Table 2. The product oil was subjected to atmospheric distillation, and a 255-265°C fraction was collected. The purity of dimethylnaphthalenes was 65%.

Table 1

| | Desulfurized Kerosene Fraction | Raffinate |
|---|---|---|
| Specific Gravity (15/4°C) | 0.7926 | 0.8026 |
| Viscosity (cSt (mm$^2$/s),30°C) | 1.420 | 1.738 |
| Total Nitrogen-Content (ppm) | 0.5 or less | 0.5 or less |
| Sulfur Content (ppm) | 0.1 or less | 0.1 or less |
| Water Content (ppm) | 30 | 36 |
| Composition (vol%) | | |
| Saturated | 93.5 | 88.1 |
| Unsaturated | 0.5 | 0.7 |
| Aromatic | 11.2 | 11.2 |
| Distillation Properties | | |
| Initial Distillation Point (°C) | 181.5 | 194.5 |
| 50% Distillation Point (°C) | 210.5 | 211.0 |
| 95% Distillation Point (°C) | 243.0 | 242.5 |
| End Point (°C) | 256.0 | 257.5 |
| Dimethylnaphthalne Content (wt%) | 0 | 0 |

4

Table 2

| | | Example 1 | Example 2 | Comparative Example 1 |
|---|---|---|---|---|
| | Type of Oil | Raffinate | Raffinate | Desulfurized oil |
| Condition | Temperature (°C) | 470 | 490 | 490 |
| | Pressure (kg/cm2G) [*98,07 kPa] | 10 | 10 | 5 |
| | LHSV (h⁻¹) | 0.8 | 0.8 | 0.8 |
| | H₂/Oil (molar ratio) | 3 | 3 | 2 |
| Properties of Product Oil | Specific Gravity (15/4°C) | 0.8514 | 0.8621 | 0.8569 |
| | Viscosity (cSt, 30°C) [mm²/s] | 1.043 | 0.9828 | 1.134 |
| | Total Nitrogen Content (ppm) | 0.5 or less | 0.5 or less | 0.5 or less |
| | Sulfur Content (ppm) | 0.1 or less | 0.1 or less | 0.1 or less |
| | Composition(%) Saturated | 36.0 | 30.4 | 35.6 |
| | Unsaturated | 0 | 0 | 0 |
| | Aromatic | 64.0 | 69.6 | 64.4 |
| | Distillation Properties Initial Distillation Point (°C) | 47.5 | 45.0 | 51.5 |
| | 50% Distillation Point (°C) | 198.0 | 198.0 | 204.5 |
| | 95% Distillation Point (°C) | 287.0 | 302.0 | 286.0 |
| | End Point (°C) | 310.5 | 308.0 | 311.0 |
| Composition | 2,6-Dimethylnaphthalene | 1.48 | 2.09 | 0.77 |
| | 2,7-Dimethylnaphthalene | 1.24 | 1.55 | 0.77 |
| | 1,6-Dimethylnaphthalene | 2.35 | 2.81 | 1.91 |
| | 1,2 to 1,5-Dimethylnaphthalenes | 1.67 | 4.00 | 2.23 |
| | 1,8 and 2,3-Dimethylnaphthalenes | 1.85 | 2.87 | 1.59 |
| | Total of Dimethylnaphthalenes | 8.59 | 13.32 | 7.27 |

EXAMPLES 3 TO 5, AND COMPARATIVE EXAMPLE 2

Raffinates in which the percent recovery of normal paraffins was adjusted to 50% by weight and 70% by weight by adding the normal paraffins recovered in each of Examples 1 and 2 to the raffinate used in each of Example 1 and 2, and for comparison, the desulfurized kerosene used in Comparative Example 1 were subjected to reforming reaction by the use of a catalytic reforming catalyst comprising an alumina carrier having supported thereon 0.2% by weight of platinum and under the conditions of pressure of 25 kg/cm$^2$ (2452 kPa), temperature of 490°C, LHSV of 0.8 h$^{-1}$, and hydrogen/oil molar ratio of 6. Properties and composition of the product oil are shown in Table 3.

Table 3

| | | Example 3 | Example 4 | Example 5 | Comparative Example 2 |
|---|---|---|---|---|---|
| | Type of Oil and Percent Recovery (wt%) | Raffinate 50 | Raffinate 70 | Raffinate 90 | Desulfurized Oil 0 |
| Properties of Product Oil | Specific Gravity (15/4°C) | 0.8311 | 0.8321 | 0.8343 | 0.8281 |
| | Viscosity (cSt, 30°C) [mm2/s] | 0.8494 | 0.8498 | 0.8513 | 0.8489 |
| | Total Nitrogen Content (ppm) | 0.5 or less | 0.5 or less | 0.5 or less | 0.5 or less |
| | Sulfur Content (ppm) | 0.1 or less | 0.1 or less | 0.1 or less | 0.1 or less |
| | Distillation Properties | | | | |
| | Initial Distillation Point (°C) | 40.5 | 40.0 | 41.0 | 50.0 |
| | 50% Distillation Point (°C) | 189.0 | 189.0 | 190.0 | 204.0 |
| | 95% Distillation Point (°C) | 290.0 | 290.5 | 297.5 | 286.5 |
| | End Point (°C) | 309.0 | 306.0 | 306.5 | 307.5 |
| Composition | 2,6-Dimethylnaphthalene | 1.54 | 1.76 | 1.81 | 1.17 |
| | 2,7-Dimethylnaphthalene | 1.56 | 1.80 | 1.84 | 1.21 |
| | 1,6-Dimethylnaphthalene | 1.54 | 2.61 | 2.63 | 1.21 |
| | 1,2 to 1,5-Dimethylnaphthalenes | 2.53 | 3.46 | 3.55 | 1.82 |
| | 1,8 and 2,3-Dimethylnaphthalenes | 2.86 | 2.57 | 2.59 | 2.52 |
| | Total of Dimethylnaphthalenes | 10.03 | 12.20 | 12.42 | 7.93 |

As apparent from the foregoing results, by reforming reaction of a raffinate resulting from the recovery of normal paraffins from a desulfurized kerosene fraction, dimethylnaphthalenes can be obtained in the concentration of about 1.5 times that from the desulfurized kerosene fraction.

## Claims

1. A process for producing dimethylnaphthalenes which comprises subjecting a raffinate resulting from the recovery of normal paraffins from a hydrodesulfurized kerosene fraction (i.e. the normal paraffin depleted fraction) to reforming reaction and then recovering dimethylnaphthalenes from the product oil.

2. The process as claimed in Claim 1, wherein the raffinate is a raffinate resulting from the recovery of at least 50% by weight of normal paraffins from a hydrodesulfurized kerosene fraction.

3. The process as claimed in Claim 1, wherein the reforming reaction is carried out by the use of a catalyst prepared by supporting platinum alone or in combination with rhenium, germanium, tin, iridium, or rhutenium on an alumina carrier and under the conditions of temperature range of from 400 to 550°C, pressure range of from 1 to 100 kg/cm$^2$ (98 to 9807 kPa), liquid hourly space velocity range of from 0.1 to 3 h$^{-1}$ and hydrogen/oil molar ratio range of from 0.5 to 20.

4. The process as claimed in Claim 1, wherein the reforming reaction is carried out by the use of a zeolite, or crystalline aluminosilicate, silica, alumina, zirconia, titania, chromia, solid phosphoric acid, or oxides of indium, lanthanum, manganese, cerium or tin, or acidic refractories containing a mixture of two or more thereof, or a catalyst prepared by containing therein or supporting thereon a metal selected from platinum, palladium, and rhenium under the conditions of temperature range of from 250 to 700°C, pressure range of from 1 to 100 kg/cm$^2$ (98 to 9807 kPa), liquid hourly space velocity range of from 0.1 to 20 h$^{-1}$, and hydrogen/oil molar ratio range of from 0.5 to 20.

5. The process as claimed in Claim 3, wherein the catalyst for the reforming reaction is a catalyst for catalytic reforming of naphtha.

6. The process as claimed in Claim 1, wherein the recovery of dimethylnaphthalenes is carried out by distillation, solvent extraction, conventional crystallization, or high-pressure crystallization, or a combination thereof.

7. The process as claimed in Claim 6, wherein the recovery of dimethylnaphthalenes is carried out by distillation to collect a 255-270°C fraction.

8. The process as claimed in Claim 1, wherein the recovery of dimethylnaphthalenes is finally the separation and recovery of 2,6- and 2,7-dimethylnaphthalenes.

9. The process as claimed in Claim 1 wherein the hydrodesulfurized kerosene fraction is a kerosene fraction with reduced sulfur and nitrogen contents to 50 ppm or less.

10. A process as in claim 1, wherein 2,6- and 2,7-dimethylnaphthalenes are recovered from the dimethylnaphthalenes by any one or more methods of adsorption separation, crystallization, or separation forming a complex compound; and then the recovery residue is isomerized into 2,6- and 2,7-dimethylnaphthalenes.

## Patentansprüche

1. Verfahren zur Herstellung von Dimethylnaphthalinen, welches umfaßt: Unterwerfen eines Raffinats, das aus der Rückgewinnung normaler Paraffine aus einer hydroentschwefelten Kerosinfraktion erhalten wird, (d.h. der von normalem Paraffin abgereicherten Fraktion), erhalten wird, einem Reformierungsverfahren und anschließendes Gewinnen der Dimethylnaphthaline aus dem Ölprodukt.

2. Verfahren nach Anspruch 1, worin das Raffinat ein Raffinat ist, das durch Rückgewinnung von mindestens 50 Gew.-% der normalen Paraffine aus einer hydroentschwefelten Kerosinfraktion erhalten wird.

**3.** Verfahren nach Anspruch 3, worin die Reformierungsreaktion unter Verwendung eines Katalysators, der durch Auftragung von Platin allein oder in Kombination mit Rhenium, Germanium, Zinn, Iridium oder Rhutenium auf einen Aluminiumoxidträger hergestellt wird, und unter den Bedingungen eines Temperaturbereiches von 400 bis 550°C, eines Druckbereiches von 1 bis 100 kg/cm$^2$ (98 bis 9807 kPa), einer Flüssigvolumen-Geschwindigkeit pro Stunde im Bereich von 0,1 bis 3h$^{-1}$ und eines Wasserstoff/Öl - Mol-Verhältnisses im Bereich von 0,5 bis 20 durchgeführt wird.

**4.** Verfahren nach Anspruch 1, worin die Reformierungsreaktion unter Verwendung eines Zeoliten oder kristallinem Aluminiumsilikats, Siliziumoxids, Aluminiumoxids, Zirkoniumoxids, Titaniumoxids, Chromoxids, fester Phosphorsäure oder Oxiden von Indium, Lanthan, Mangan, Cer oder Zinn, oder sauren feuerfesten Materialien, die eine Mischung von zwei oder mehr davon enthalten, oder einen Katalysator, der ein Metall, ausgewählt aus Platin, Palladium und Rhenium enthält, oder auf dem ein solches Material aufgebracht ist, unter den Bedinungen eines Temperaturbereiches von 250 bis 700°C, eines Druckbereiches von 1 bis 100 kg/cm$^2$ (98 bis 9807 kPa), einer Flüssigvolumengeschwindigkeit pro Stunde im Bereich von 0,1 bis 20 h$^{-1}$, und eines Wasserstoff/Öl - Mol-Verhältnisses im Bereich von 0,5 bis 20 unterworfen wird.

**5.** Verfahren nach Anspruch 3, worin der Katalysator für die Reformierungsreaktion ein Katalysator für die katalytische Reformierung von Naphtha ist.

**6.** Verfahren nach Anspruch 1, worin die Gewinnung von Dimethylnaphthalinen durch Destillation, Lösungsextraktion, übliche Kristallisation oder Hochdruckkristallisation oder eine Kombination davon, durchgeführt wird.

**7.** Verfahren nach Anspruch 6, worin die Gewinnung von Dimethylnaphthalinen durch Destillation unter Sammlung einer 255 - 270°C -Fraktion durchgeführt wird.

**8.** Verfahren nach Anspruch 1, worin die Gewinnung von Dimethylnaphthalinen durch Trennung und Gewinnung von 2,6- und 2,7-Dimethylnaphthalinen abgeschlossen wird.

**9.** Verfahren nach Anspruch 1, worin die hydroentschwefelte Kerosinfraktion eine Kerosinfraktion mit auf 50 ppm oder weniger verminderten Schwefel- und Stickstoffgehalten ist.

**10.** Verfahren nach Anspruch 1, worin 2,6- und 2,7-Dimethylnaphthaline aus den Dimethylnaphthalinen nach einem oder mehreren Verfahren der Adsorptionstrennung, Kristallisation oder Trennung unter Bildung einer Komplexverbindung gewonnen werden, und anschließend die Rückstände zu 2,6- und 2,7-Dimethylnaphthalinen isomerisiert werden.

**Revendications**

**1.** Procédé de production de diméthylnaphtalènes, qui comporte le fait de soumettre à une réaction de reformage un raffinat résultant de la récupération de paraffines normales à partir d'une fraction de kérosène ayant subi une hydrodésulfuration (c'est-à-dire une fraction appauvrie en paraffines normales), puis la récupération des diméthylnaphtalènes à partir de l'huile obtenue.

**2.** Procédé conforme à la revendication 1, dans lequel le raffinat est un raffinat résultant de la récupération d'au moins 50 % en poids des paraffines normales à partir d'une fraction de kérosène ayant subi une hydrodésulfuration.

**3.** Procédé conforme à la revendication 1, dans lequel la réaction de reformage est effectuée à l'aide d'un catalyseur préparé par dépôt de platine, seul ou combiné à du rhénium, du germanium, de l'étain, de l'iridium ou du rhuténium, sur un support d'alumine, et dans les conditions suivantes : température située dans l'intervalle allant de 400 à 550°C, pression située dans l'intervalle allant de 1 à 100 kg/cm$^2$ (98 à 9807 kPa), vitesse spatiale horaire du liquide située dans l'intervalle allant de 0,1 à 3 h$^{-1}$, et rapport molaire hydrogène/huile situé dans l'intervalle allant de 0,5 à 20.

**4.** Procédé conforme à la revendication 1, dans lequel la réaction de reformage est effectuée à l'aide d'une zéolithe, ou d'un aluminosilicate cristallin, de silice, d'alumine, de zircone, d'oxyde de titane, d'oxyde de chrome, d'acide phosphorique solide, ou d'oxyde d'indium, de lanthane, de manganèse, de cérium ou d'étain, ou de matériaux réfractaires à caractère acide, contenant un mélange de deux de ces composés ou plus, ou à l'aide d'un catalyseur que l'on a préparé en y incorporant ou en y déposant un métal choisi parmi du platine, du palladium et du rhénium, et dans les conditions suivantes : température située dans l'intervalle allant de 250 à 700°C, pression située dans l'intervalle allant de 1 à 100 kg/cm$^2$ (98 à 9807 kPa), vitesse spatiale horaire du liquide située dans l'intervalle allant de 0,1 à 20 h$^{-1}$, et rapport molaire hydrogène/huile situé dans l'intervalle allant de 0,5 à 20.

5. Procédé conforme à la revendication 3, dans lequel le catalyseur employé pour la réaction de reformage est un catalyseur utilisé pour le reformage catalytique du naphta.

6. Procédé conforme à la revendication 1, dans lequel la récupération des diméthylnaphtalènes est effectuée par distillation, extraction par solvant, cristallisation classique ou cristallisation sous haute pression, ou par une combinaison de ces méthodes.

7. Procédé conforme à la revendication 6, dans lequel on effectue la récupération des diméthylnaphtalènes par distillation, en recueillant la fraction passant à 255 - 270°C.

8. Procédé conforme à la revendication 1, dans lequel la récupération des diméthylnaphtalènes comporte enfin la séparation et la récupération des 2,6- et 2,7-diméthylnaphtalènes.

9. Procédé conforme à la revendication 1, dans lequel la fraction de kérosène ayant subi une hydrodésulfuration est une fraction de kérosène dont les teneurs en soufre et en azote ont été réduites à 50 ppm ou moins.

10. Procédé conforme à la revendication 1, dans lequel on récupère les 2,6- et 2,7-diméthylnaphtalènes, à partir des diméthylnaphtalènes, selon l'une quelconque ou plusieurs des méthodes de séparation par adsorption, par cristallisation ou par formation d'un composé complexe, et le résidu de récupération est ensuite isomérisé en 2,6- et 2,7-diméthylnaphtalènes.